(19) Europäisches Patentamt
European Patent Office
Office européen des brevets

(11) Publication number: **0 006 757**
**A2**

# (12) EUROPEAN PATENT APPLICATION

(21) Application number: 79301240.2

(22) Date of filing: 26.06.79

(51) Int. Cl.3: **A 61 K 6/00**, A 61 K 6/06

(30) Priority: 26.06.78 IE 1266/78

(43) Date of publication of application: 09.01.80
Bulletin 80/1

(84) Designated Contracting States: BE DE FR GB IT

(71) Applicant: O'Sullivan, Denis J., 6 Greenfield Crescent, Donnybrook, Dublin 4 (IE)

(72) Inventor: O'Sullivan, Denis J., 6 Greenfield Crescent, Donnybrook, Dublin 4 (IE)

(74) Representative: Baillie, Iain Cameron et al, c/o Ladas, Parry, von Gehr. Goldsmith & Deschamps Blumenstrasse 48, D-8000 München 2 (DE)

(54) **Dental mixing tool for catalyzed two-part compositions and method for catalyzing said compositions.**

(57) An elongate dental tool, one end of which is charged with a catalyst in a binder material, is used to mix the co-reactive parts of a reactive mixture, e.g., a dental filling composition. Preferably, the charged catalyst is one part of a two-part catalyst system, and the binder is a monomeric or prepolymeric component of the reactive mixture.

EP 0 006 757 A2

DENTAL MIXING TOOL FOR CATALYZED TWO-PART COMPOSITIONS

TITLE MODIFIED
see front page

This invention relates to a dental tool for hand-mixing catalyzed compositions immediately prior to their use. It is particularly useful in connection with dental filling compositions and will be described in that context. It is to be appreciated, however, that the scope and spirit of the invention extend beyond the field of dental filling compositions.

It is known to present a catalyzed dental filling composition as a pair of pastes which have to be mixed together before application to a cavity. For reasons connected with their manufacture, it is not possible to color the members of the pair identically; the mixture of the two is accordingly of a third color, and the same is true of the cured composition, which makes it difficult to match the filling color to the patients' teeth.

It is also known to present a catalyzed dental filling composition as a paste and a liquid, and to require that a small volume of the liquid be added to and mixed with the paste to effect cure. Thus, with a two-part catalyst system comprising, for example, an amine and a peroxide, one part is present in the paste and the other in the liquid. It is difficult, however, to meter the small quantities of liquid involved, and it is difficult to produce a reliably leakproof applicator. Moreover the mixture of paste and liquid, freshly made, tends to a viscosity of the order to 100,000 poise, which is undesirably low (200,000 - 400,000 poise is easier to handle and retains its shape better on molding), and even this is only achieved by using a paste of such very

high viscosity (500,000 - 900,000 poise) that it is difficult to mix with the liquid.

It is also known to present a catalyzed dental filling composition as a paste and a small sheet of paper which contains, in coated or impregnated form, sufficient catalyst or catalyst component for a single dental filling. The paper sheets are supplied as a book-like pad with leaves which can be torn off, and which are discarded after use. A charge of paste, sufficient for one filling, is placed on a sheet of paper and kneaded or worked to contact it with the catalyst. This presentation of the dental composition has at least three drawbacks, viz.

a) it is necessary but difficult and time-consuming to spread and mix the paste over the entire surface of one side of the paper;

b) owing to the normally abrasive nature of the paste, an undesirably high proportion of paper fibers is incorporated into the mixture, leading to mechanical weakness in the cured filling;

c) curing results are erratic: some fillings fail to cure satisfactorily despite careful handling.

It is known further to present a catalyzed dental filling composition as a paste and a separate, microencapsulated catalyst to be mixed with the paste using sufficient shear action in the mixing to rupture the microcapsules, thereby liberating the catalyst. A drawback to this presentation is that a quantity of capsule coating remains in the mixture, producing mechanical weakness in the cured filling.

The invention comprises an elongate tool having two ends, one of said ends adapted to function as a handle, the other of said ends having adhered thereto a catalyst-containing composition comprising a firm, nontacky binder material containing said catalyst, said tool adapted for mixing coreactive materials and thereby subjecting them to the catalytic influence of said catalyst.

The binder may itself be one of the coreactive

materials, or it may be an additional coreactive material. It may also be a nonreactive material which is, however, compatible with the reactive system.

The catalyst may itself have catalytic activity, and/or it may act as a cocatalyst or accelerator for other catalytic materials in the reactive system.

The present invention provides the amelioration or removal of some or all of the above-recited drawbacks. The invention improves catalyzed dental filling compositions by making them simple to manipulate, enabling them to be accurately color-matched, and rendering them reliable in curing, mechanically sound when cured, and substantially devoid of contaminants.

The present invention, therefore, provides an elongate tool for hand-mixing catalyzed dental filling compositions immediately prior to their use, whereof one end is charged with an effective amount of an appropriate catalyst in a binder, the other end being adapted to be held by hand.

The elongate tool may comprise, for example, a simple mixing rod, a spatula, a spoon or a probe. It may be made of glass, ceramics, plastics, metal, wood or other suitable material or combination of materials. For example, it may have a working end of glass and a handle of plastic.

Where a reactive composition involves a multicomponent catalyst system, the expression "an appropriate catalyst" used above is to be understood as including at least one essential component of such system. The invention is not limited to any particular catalyst, the selection of the catalyst being within the skill of the art. If the catalyst system is a multi-part system, more than one of such parts of such system could be contained in the binder; provided that the catalyst parts are selected so as not to interfere significantly with the storage stability of the charged spatula or exhibit other adverse effects. The physical form of the catalyst, e.g., whether dissolved in the binder or in particulate form

dispersed in the binder, is not considered to be critical, provided that the catalyst remains able to exert the necessary catalytic effect when the tool is used for mixing.

Some dental filling composition catalyst may adhere directly to the working end of a mixing tool, for example, where the catalyst is applied as a solution in a volatile solvent which is then allowed to dry. Other catalysts which are, for example, soft solids or pastes may be caused to remain on the tool by making the working end of the tool of a rough texture, for example fibrous, spongy or pumice-like. Still other catalysts may require an adhesive to ensure adequate fixation to the tool. For these latter catalysts, a preferred embodiment of the invention provides that they be affixed to the working end of the tool with the aid of an adhesive which is compatible with the dental filling composition. More preferably, the adhesive is selected from the list of the ordinary ingredients of the composition.

In a particularly preferred embodiment, the dental filling composition uses a two-part catalyst system. Such a system may comprise an amine in cooperation with a peroxide. One of the two parts, e.g., the amine or the peroxide, is applied to the mixing tool with the aid of an adhesive which comprises a small amount of the same monomer or prepolymer as is used in making up the paste which forms the main bulk of the resin of the dental filling composition. The other of the two parts, e.g., the peroxide or the amine, is incorporated in said paste. A mixture of the catalyst component and the monomer or prepolymer is dissolved in a volatile solvent, and the mixing tool tip is dipped in the resulting solution, removed and the solvent is allowed to evaporate. The residue of monomer or prepolymer dries to a firm, preferably nontacky consistency, and acts as an adhesive or binder to fix the catalyst component on the tool. Surprisingly, said residue does not polymerize, however, until the action of

stirring the paste with the tool brings the two catalyst components together in the presence of the liquid or paste monomer- or prepolymer-containing part of the composition.

The invention will now be described in more specific detail in the following illustrative example.

EXAMPLE

A two-part dental filling composition was prepared by mixing the following ingredients, expressed as parts by weight:

Part 1

| | | |
|---|---|---|
| 1. | Monomer A | 14 |
| 2. | Monomer B | 8 |
| 3. | Methacrylic acid | 1 |
| 4. | Methacryloxy trimethoxysilane | 0.66 |
| 5. | Naphthoquinone | 0.3 |
| 6. | N,N-dimethyl-p-toluidine | 0.5 |
| 7. | Hydroxypropyl methacrylate | 14 |
| 8. | "Uniflex" 330 | 10 |
| 9. | Glass Powder, q.s.ad. | 100 |

Ingredients 1 and 2 are described in more detail below. Ingredients 3 and 4 are adhesion promoters; 5 is a stabilizer; 6 is one part of a two-part catalyst system; 7 is a viscosity regulator; 8 is another viscosity regulator, a proprietary product of Union Camp Corporation, Wayne, New Jersey, U.S.A., which comprises poly(butylene sebacate) ("Uniflex" is a trademark); 9 is a mechanical extender and toughener as used in conventional dental filling compositions.

Part 2

| | | |
|---|---|---|
| 10. | Monomer A | 8 g. |
| 11. | Benzoyl peroxide | 2 g. |
| 12. | p-Benzoquinone | 0.037 mg. |
| 13. | Chloroform | 41.7 g. |

Ingredients 1 and 2, otherwise monomers A and B, are urethane acrylates having molecular structures as follows:

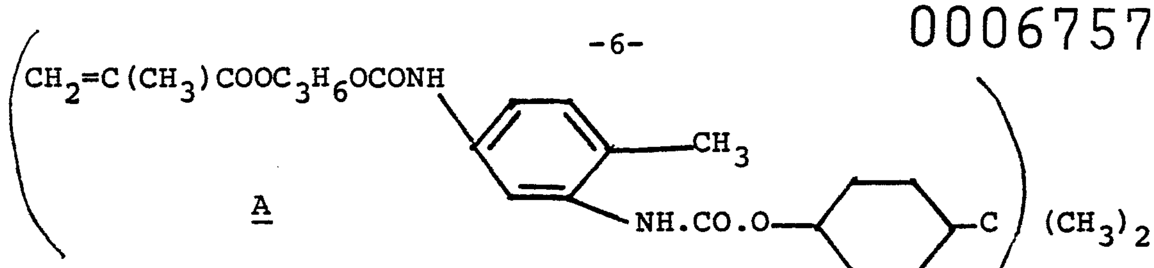

A

and

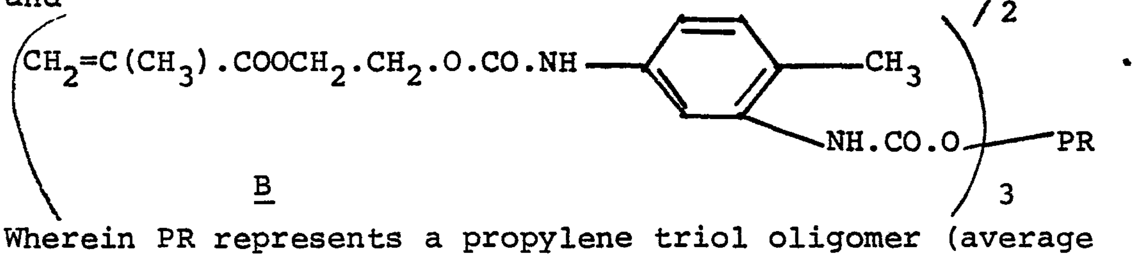

B

Wherein PR represents a propylene triol oligomer (average molecular weight 2500) devoid of its three hydroxyl groups. They are described, along with other prepolymers suitable for use with the mixing tool of the invention, in co-pending Patent Application No. 1580/72, which also gives general methods for their preparation, and lists of starting components for use in the methods. The disclosure of this is incorporated herein by reference.

A solution of the ingredients 10, 11 and 12 was made in the chloroform. A number of clean, dry spatulas of polypropylene plastic, each 120 mm long x 4 mm wide x 2 mm in thickness, was provided. Each spatula was charged by dipping one end thereof into the chloroform solution to a depth of 10 mm, withdrawing the spatula and allowing the dip-coated charge to dry in air at room temperature for 3-4 hours. The charges dried to an average additional weight of 9.3 mg per spatula.

In the second part of the composition, ingredient 12 is the second part of a two-part catalyst system. Ingredient 10 is used as an adhesive to fix ingredient 12 to the spatula. It is intended to undergo polymerization later, together with ingredient 1 of the first part, with which, being identical, it is, of course, completely compatible.

200 Mg of the first part was mixed on a mixing tile by means of one of the prepared spatulas, for 30 seconds, using the charged end to do the mixing. The resulting paste set hard in 2 minutes 10 seconds.

Another 200 mg of the first part, similarly mixed and applied to a tooth cavity, solidified within a similar interval to an acceptable dental filling.

Further spatulas were charged by dipping them into a solution similar to part 2 but containing either more chloroform or less. In this way, spatulas bearing charges that ranged from 6.2 mg to 14 mg. in weight were prepared. When used, one at a time, to mix 200 mg. aliquots of part 1 for 30 seconds as described above, they gave cure speeds which ranged from 5 minutes 30 seconds to 0 minutes 40 seconds, in approximate proportion to the amount of the charge. It will be observed that the ratio of catalyst to binder on the spatula is essentially a matter of choice, depending upon cure speed, and related properties, desired. Likewise, the ratio of catalyst contained on the spatula to the remainder of the total reactive mixture will also be a matter of choice within the skill of the art to determine.

What we claim is:

1. An elongate dental tool having two ends, one of said ends adapted to function as a handle, the other of said ends having adhered thereto a catalyst-containing composition comprising a firm, nontacky binder material containing said catalyst, said tool adapted for mixing coreactive materials and thereby subjecting them to the catalytic influence of said catalyst.

2. A tool according to claim 1, wherein the binder is one of the coreactive materials.

3. A tool according to claim 1, wherein the catalyst is dissolved in the binder.

4. A tool according to claim 1, wherein the catalyst is dispersed in the binder.

5. A tool according to claim 1, wherein the binder is a nonreactive material.

6. A method for catalyzing a reactive mixture comprising mixing said mixture with the tool of claim 1.